**Europäisches Patentamt**

**European Patent Office**

**Office européen des brevets**

(19)

(11) Veröffentlichungsnummer: **0 079 493**
**B1**

(12) **EUROPÄISCHE PATENTSCHRIFT**

(45) Veröffentlichungstag der Patentschrift:
19.09.84

(51) Int. Cl.³: **C 07 C 149/20,** C 07 C 148/00

(21) Anmeldenummer: **82109868.8**

(22) Anmeldetag: **26.10.82**

(54) Verfahren zur Herstellung von Auromercaptobernsteinsäure.

(30) Priorität: **29.10.81 DE 3142944**

(43) Veröffentlichungstag der Anmeldung:
**25.05.83 Patentblatt 83/21**

(45) Bekanntmachung des Hinweises auf die Patenterteilung:
**19.09.84 Patentblatt 84/38**

(84) Benannte Vertragsstaaten:
**AT BE CH DE FR GB IT LI LU NL SE**

(56) Entgegenhaltungen:
**DE - A - 1 568 063**
**DE - C - 949 055**
**US - A - 2 370 592**

**Chemical Abstracts Band 45, Nr. 13, 10. Juli 1951,
Columbus, Ohio, USA E.E. MOORE et al. "Association of
aurothiocarboxy acids and their salts" Spalte 5879**

(73) Patentinhaber: **Byk Gulden Lomberg Chemische Fabrik
GmbH, Byk-Gulden-Strasse 2, D-7750 Konstanz (DE)**

(72) Erfinder: **Haas, Dieter, Dr., Münsterplatz 6,
D-7752 Reichenau (DE)**
Erfinder: **Rapp, Erich, Dr., Pfarrer-Braun-Strasse 6,
D-7760 Radolfzell 17 (DE)**
Erfinder: **Reiter, Winfried, Dr., Emmishofer Strasse 18,
D-7750 Konstanz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Auromercaptobernsteinsäure.

Auromercaptobernsteinsäure ist ein in der Therapie der progredient chronischen Polyarthritis seit längerem eingesetzter Wirkstoff, der in der Regel als Natriumsalzlösung intramuskulär appliziert wird.

Es sind mehrere Verfahren zur Herstellung von Auromercaptobernsteinsäure (Aurothioäpfelsäure) bekannt, bei denen Au(I)-Salze in wäßriger Lösung mit Mercaptobernsteinsäure oder ihrem Natriumsalz umgesetzt werden. In der DE-OS 1 568 063 wird die Herstellung von Thiogold-(I)-Verbindungen durch Umsetzung von Goldfulminat mit entsprechenden organischen Thioverbindungen, u. a. Thioäpfelsäure, in wäßriger Lösung beschrieben. Goldfulminat muß dabei jeweils frisch durch Reaktion einer Gold(III)-Verbindung mit wäßrigem Ammoniak hergestellt werden. Gemäß US-PS 1 994 213 wird das Natriumsalz der Auromercaptobernsteinsäure durch Umsetzung von Natriumthiomalat mit Gold(I)-iodid hergestellt. Wegen ihrer Instabilität muß die Ausgangsverbindung AuJ jeweils frisch hergestellt werden. Das gewünschte Endprodukt wird erst nach mehrmaligem aufwendigem Umfällen erhalten. Die Nachteile dieses Verfahrens werden z. B. in der US-PS 2 370 592 (Spalte 1, Zeilen 28 bis 37) abgehandelt. In der US-PS 2 352 124 wird die Fällung von Calciumaurothiomalat durch doppelte Umsetzung von Natriumaurothiomalat mit Calciumchlorid in wäßriger Lösung beschrieben. Gemäß der US-PS 2 370 592 wird Aurothiobernsteinsäure durch Umsetzung von Gold(I)-cyanid oder Cyanoauraten(I) mit Mercaptobernsteinsäure in wäßriger Lösung erhalten. Die wäßrige Suspension muß zuerst vollständig eingedampft werden, bevor Nebenprodukte durch Waschen entfernt werden können. Wegen des freiwerdenden HCN sind besondere Vorsichtsmaßnahmen erforderlich. Die Ausgangsverbindung K[Au(CN)$_2$] wird durch Umsetzung von H[AuCl$_4$] mit KCN in Gegenwart eines Reduktionsmittels hergestellt. In der US-PS 2 370 592 wird die Umsetzung von Gold(I)-cyanid oder Cyanoauraten(I) mit Verbindungen R—SH, in denen R einen Arylrest darstellt, beansprucht.

Die vorstehend beschriebenen Verfahren sind wegen der damit verbundenen Eindampf-, Extraktions- oder Fällungsoperationen zeit- und arbeitsaufwendig. Die wäßrigen Lösungen der Auromercaptobernsteinsäure sind lichtempfindlich, beim Arbeiten in Verdampfern oder mit Rührern kommt es durch den Kontakt mit Dichtungen oder Metallteilen oft zur Zersetzung (Goldabscheidung). Es wurde nun ein Verfahren zur Herstellung von Auromercaptobernsteinsäure gefunden, das die Nachteile der bisher bekannten Verfahren vermeidet.

Gegenstand der Erfindung ist ein Verfahren zur Herstellung von Auromercaptobernsteinsäure, dadurch gekennzeichnet, daß man Gold(III)-chlorid oder Tetrachlorogold(III)-säure mit Mercaptobernsteinsäure in aliphatischen Ketonen mit 3 bis 10 Kohlenstoffatomen umsetzt.

Die Goldverbindungen können wasserfrei oder in Form ihrer Hydrate eingesetzt werden, z. B. als AuCl$_3$, AuCl$_3$ · 3 H$_2$O oder handelsübliches HAuCl$_4$ · nH$_2$O (Fa. Degussa: n ~ 3,5, ca. 50% Au).

Die aliphatischen Ketone können verzweigt oder geradkettig sein. Beispielsweise seien genannt Aceton, 2-Butanon, Isobutylmethylketon, Ethylisopropylketon, 2-Hexanon, 3-Hexanon, 2-Octanon, 2-Decanon. Bevorzugt sind Ketone mit 4 bis 6 Kohlenstoffatomen, besonders bevorzugt Isobutylmethylketon.

Die Reaktionszeiten für die Umsetzung im technischen Maßstab liegen im Bereich von 1 bis 40 Stunden, vorzugsweise bis 20 Stunden. Die Reaktion kann bei Temperaturen von —20°C bis zur Siedetemperatur des aliphalischen Ketons durchgeführt werden. Bevorzugt wird die Reaktion bei Raumtemperatur (20—30°C) ausgeführt, woran ein kurzfristiges (unter einer Stunde) Erwärmen auf Temperaturen um den Siedepunkt des Lösungsmittels angeschlossen wird. Die Isolierung und Aufarbeitung des Endproduktes erfolgt nach dem Fachmann bekannten Methoden, wie Absaugen und Auswaschen.

Das erfindungsgemäße Verfahren liefert das Endprodukt mit hoher Ausbeute und in gleichmäßiger und hoher Qualität. Infolge des geringen Aufwandes an Lösungsmittel, der Rückgewinnbarkeit und Wiedereinsetzbarkeit des Lösungsmittels und der kurzen Arbeitszeit ist es wirtschaftlicher als die bisher bekannten Verfahren.

Die folgenden Beispiele dienen zur Veranschaulichung der Erfindung.

### Beispiel 1

#### Lösung A

330 g gereinigte D,L-Mercaptobernsteinsäure werden in 2,5 Liter Isobutylmethylketon bei 80°C gelöst. Nach Zugabe von 15 g Aktivkohle wird 10 Minuten bei dieser Temperatur gerührt und dann in einen 4-l-Flanschkolben klar filtriert.

#### Lösung B

250 g Tetrachlorogold(III)-säure (ca. 50% Au) werden in einem Becherglas in 400 ml Isobutylmethylketon unter Rühren gelöst. Nach Zugabe von 5 g Aktivkohle wird 10 Minuten gerührt, dann filtriert und mit 100 ml Isobutylmethylketon nachgespült.

Lösung A wird auf 20°C abgekühlt. Lösung B tropft man unter Rühren innerhalb von 1 Stunde zu Lösung A unter Inertgasatmosphäre. Unter Freiwerden von Salzsäuredämpfen entsteht zunächst eine braune Suspension. Nach 18 Stunden Rühren wird die nunmehr hellgelbe Suspension 30 Minuten auf 105°C erhitzt und dann heiß über eine Glasfritte abgesaugt. Man wäscht mit 500 ml heißem Isobutylmethylketon nach. Das abgesaugte Rohprodukt wird in 1,5 Liter siedendem Ethylacetat aufgerührt und nach 10 Minuten Rühren abgesaugt. Das Produkt wird mit 500 ml kaltem Ethylacetat gewaschen. Das Aufrühren in heißem Ethylacetat mit anschließendem Absaugen wird noch einmal durchgeführt, falls im Dünnschichtchromatogramm noch Spuren von D,L-Mercaptobernsteinsäure oder von 2,2'-Dithiodibernsteinsäure festgestellt werden.

Das lösungsmittelfeuchte Produkt wird im Vakuum bei 80°C bis zur Gewichtskonstanz getrocknet. Man erhält 212 g (97,2% der Theorie) Auromercaptobernsteinsäure.

## Beispiel 2

### Lösung A

330 g g D,L-Mercaptobernsteinsäure werden in 2 Liter 2-Butanon bei 70°C gelöst. Nach Zugabe von 15 g Aktivkohle wird 10 Minuten bei dieser Temperatur gerührt und dann in einen 4-l-Flanschkolben klar filtriert.

### Lösung B

250 g Tetrachlorogold(III)-säure (ca. 50% Au) werden in einem Becherglas in 300 ml 2-Butanon unter Rühren gelöst. Nach Zugabe von 5 g Aktivkohle wird 10 Minuten gerührt, dann filtriert und mit 100 ml 2-Butanon nachgespült.

Lösung A wird auf 20°C abgekühlt. Lösung B wird analog Beispiel 1 zur Lösung A zugegeben. Nach der Zugabe wird sofort auf Rückfluß erhitzt und bei dieser Temperatur 6 Stunden gerührt. Anschließend wird heiß abgesaugt. Man wäscht den Filterrückstand zunächst mit 400 ml heißem Butanon und rührt ihn nochmals in 1,2 l 2-Butanon auf. Nach 10minütigem Erhitzen auf Rückfluß wird wieder heiß abgesaugt und mit 400 ml heißem 2-Butanon gewaschen. Durch Trocknen im Vakuum bei 80°C bis zur Gewichtskonstanz erhält man 202,1 g (92,7% d. Th.) Auromercaptobernsteinsäure.

## Beispiel 3

### Lösung A

330 g D,L-Mercaptobernsteinsäure werden in 2,5 Liter 2-Hexanon bei 100°C gelöst. Nach Zugabe von 15 g Aktivkohle wird 10 Minuten bei dieser Temperatur gerührt und dann in einen 4-l-Flanschkolben klar filtriert.

### Lösung B

192 g Gold(III)-chlorid (ca. 64,9% Gold) werden in einem Becherglas in 400 ml 2-Hexanon unter Rühren gelöst. Nach Zugabe von 5 g Aktivkohle wird 10 Minuten gerührt, dann filtriert und mit 200 ml 2-Hexanon nachgespült.

Lösung A wird auf 20°C abgekühlt. Lösung B wird analog Beispiel 1 zur Lösung A zugesetzt. Nach der Zugabe wird 36 Stunden bei 20°C gerührt; anschließend abgesaugt. Man wäscht den Filterrückstand mit 500 ml 2-Hexanon und arbeitet das Rohprodukt analog Beispiel 1 auf. Ausbeute 198,9 g (91,2% der Theorie) Auromercaptobernsteinsäure.

## Patentansprüche

1. Verfahren zur Herstellung von Auromercaptobernsteinsäure, dadurch gekennzeichnet, daß man Gold(III)-chlorid oder Tetrachlorogold(III)-säure mit Mercaptobernsteinsäure in aliphatischen Ketonen mit 3 bis 10 Kohlenstoffatomen umsetzt.

2. Verfahren nach Anspruch 1, dadurch gekennzeichnet, daß die Umsetzung in aliphatischen Ketonen mit 4 bis 6 Kohlenstoffatomen ausgeführt wird.

3. Verfahren nach Anspruch 1, dadurch gekennzeichnet, daß die Umsetzung in Isobutylmethylketon ausgeführt wird.

4. Verfahren nach einem der Ansprüche 1 bis 3, dadurch gekennzeichnet, daß die Umsetzung mit HAuCl₄ · nH₂O (Goldgehalt ca. 50%) ausgeführt wird.

5. Verfahren nach einem der Ansprüche 1 bis 4, dadurch gekennzeichnet, daß die Umsetzung zunächst bei Raumtempertur und dann bei der Siedetemperatur des aliphatischen Ketons ausgeführt wird.

## Claims

1. Process for the preparation of auromercaptosuccinic acid, characterized in that gold(III)chloride or tetrachloroauric(III) acid are reacted with mercaptosuccinic acid in an aliphatic ketone with 3 to 10 carbon atoms.

2. Process according to claim 1, charaterized in that the reactions is carried out in aliphatic ketones with 4 to 6 carbon atoms.

3. Process according to claim 1, characterized in that the reaction is carried out in isobutyl methyl ketone.

4. Process according to one of claims 1 to 3, characterized in that the reaction is carried out with HAuCl₄ · nH₂O (content of gold about 50%).

5. Process according to one of claims 1 to 4, characterized in that the reaction in the beginning is carried out at room temperature and then at the boiling point of the aliphatic ketone.

**Revendications**

1. Procédé de préparation de l'acide aurothio succinique, qui est caractérisé par le fait qu'on fait réagir chlorure aurique(III) ou acide tetra-chloro-aurique(III) avec acide thio succinique en cétones aliphatiques de 3 à 10 atomes de carbone.

2. Procédé selon la revendication 1, caractérisé en ce que la réaction est practiquée en cétones aliphatiques de 4 à 6 atomes de carbone.

3. Procédé selon la revendication 1, caractérisé en ce que la réaction est practiquée en iso-butylméthylcétone.

4. Procédé selon une des revendications 1 à 3, caractérisé en ce que la réaction est practiquée avec $HAuCl_4 \cdot nH_2O$ (contenant environ 50% d'or).

5. Procédé selon une des revendications 1 à 4, caractérisé en ce que la réaction est practiquée tout d'abord à la température ambiante et est continue au point d'ébullition de la cétone ali-phatique.